# EUROPEAN PATENT APPLICATION

(11) **EP 2 591 773 A2**
(43) Date of publication of application: **15.05.2013**
(21) Application number: 11803786.0
(22) Date of filing: 05.07.2011
(51) Int. Cl.: A61K 9/22, A61K 31/138, A61K 31/519, A61K 9/20

(54) **TIME-DELAYED SUSTAINED RELEASE PHARMACEUTICAL COMPOSITION COMPRISING DAPOXETINE FOR ORAL ADMINISTRATION**

(30) Priority: 06.07.2010 KR 20100064988
(71) Applicant: Navipharm Co, Ltd, Suwon-si, Gyeonggi-do 441-814 (KR)
(72) Inventor: LEE, Chang-Kyoo, Suwon-si Gyeonggi-do 440-705 (KR); PARK, Sang-Geun, Suwon-si Gyeonggi-do 440-300 (KR)
(74) Representative: Mounteney, Simon James
(86) International application number: PCT/KR2011/004919
(87) International publication number: WO 2012/005500

(57) **Abstract**

The present invention relates to a time-delayed sustained release pharmaceutical composition for oral administration, which comprises an immediate release phase and a prolonged sustained release phase, wherein said immediate release phase and prolonged sustained release phase respectively comprise Dapoxetine therein as an active ingredient. The pharmaceutical composition of the present invention comprises Dapoxetine, which is an agent for treating premature ejaculation, in both the immediate release phase and the prolonged sustained release phase thereof, to thereby immediately exhibit the effectiveness of the pharmaceutical composition of the present invention in order to enable a patient to achieve sexual satisfaction during the early stage of administration, as well as to reduce side effects by means of the time-delayed sustained release of the prolonged sustained release phase during the early stage of administration and enable a continuous in vivo absorption of Dapoxetines, to thereby lengthen the duration of the effectiveness of the pharmaceutical composition of the present invention. Further, agents for treating erectile dysfunction, such as sildenafil, tadalifil or the like can be added to the immediate release phase so as to allow for a coincidence of the durations of the effectiveness of a premature ejaculation treatment agent and erectile dysfunction treatment agents, even though a half-life difference exists between the two types of treatment agents, thus maximizing patient satisfaction.

## Description

### Technical field

The present invention relates to a time-delayed sustained release pharmaceutical composition for oral administration, which comprises an immediate release phase and a prolonged sustained release phase, wherein said immediate release phase and prolonged sustained release phase respectively comprise Dapoxetine therein as an active ingredient.

### Background art

It is an established fact that premature ejaculation is one of the most common sexual complaints showing persistent or recurrent symptom of early ejaculation by minimal sexual stimulation before, during or immediately after sexual intercourse, and accounting for 30-40% of American males. Conventional agents to treat premature ejaculation mainly have included local anesthetics, such as lidocaine, which has usability inconvenience and can reduce a partner's sexual satisfaction as well. Recently, however, along with the erectile dysfunction treating agents, the first premature ejaculation treating agent for oral administration has been launched into the market as a sort of Happy Drug. It is a sort of SSRI (selective serotonin reuptake inhibitor) typically used as antidepressants, of which the product name is Priligy, ingredient name is Dapoxetine, and chemical structure formula is (S)-(+)-N, N-dimethyl-1-phenyl-3-(1-Naphthalenyloxy)-propaneamine or (S)-(+)-N, N-dimethyl-α-[2-(1-Naphthalenyloxy) ethyl-benzenemethaneamine (Chemical formula 1).

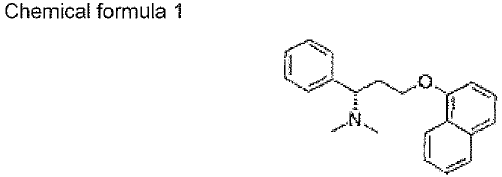

Dapoxetine has the characteristics of (1) Tmax: about 1 hour, and (2) Half Life: approximately 1.4 hours, and has an advantage that it has few side-effects due to the in vivo accumulation despite of repeated administration because the exhibition of the effectiveness of Dapoxetine is quicker than other SSRI and the drug elimination rate in the serum is also fast. Therefore, Dapoxetine needs to be taken within 1-3 hours before intercourse to get appropriate medicinal effect, but the effect does not last long because the half life is short and most of the drug in the blood is lost within 24 hours after dosing as can be seen in the plasma concentration diagram, which needs to be improved.

On the other hand, in many cases, the premature ejaculation patients also have erectile dysfunction symptoms, but only Phosphodiesterase-5 (PDE-5) inhibitors such as Viagra have mainly been prescribed so far to the premature ejaculation patients regardless of the erectile dysfunction. Major PDE-5 inhibitors have characteristics as follows.

(1) Sildenafil-Tmax: 1 hour

Half Life: 3-4 hours

(2) Tadalafil-Tmax: 2 hours

Half Life: 17.5 hours

(3) Vardenafil-Tmax: 0.7 hour

Half Life: 4-5 hours

(4) Udenafil-Tmax: 1 hour

Half Life: 7-12 hours

(5) Avanafil-Tmax: 0.3-0.5 hour

Half Life: 5-11 hours

As shown above, most of the PDE-5 inhibitors have short Tmax, which means that the exhibition of the medicinal effects is fast, and have long half life, showing extended duration of the medicinal effect. Premature ejaculation treatment agents and erectile dysfunction treatment agents has similar Tmax of about one hour, so the patients can be sexually satisfied in the early hours after taking both agents at the same time, but the effect cannot be continued after a certain time of the administration of the agents due to the difference of their half life, which is another need for the improvement.

### Disclosure of Invention

### Technical Problem

The purpose of the present invention is to provide a time-delayed sustained release pharmaceutical composition for oral administration, which comprises an immediate release phase and a prolonged sustained release phase, wherein said immediate release phase and prolonged sustained release phase respectively comprise Dapoxetine therein.

### Technical solution

In order to address the aforementioned issues, the present invention offers a time-delayed sustained release pharmaceutical composition for oral administration, which comprises an immediate release phase and a prolonged sustained release phase, wherein said immediate release phase and prolonged sustained release phase respectively comprise Dapoxetine therein.

The pharmaceutical composition of the present invention is optimized by the first pulse, which was planned for the active ingredient is released immediately from the immediate release phase after drug administration to immediately express the medicinal effects by in vivo absorption; and by the second pulse of the elution profile where the active ingredient is additionally released from the sustained release phase after a certain amount of time has elapsed to reduce the initial side effects, as well as to ensure that long-term expression of the efficacy by continued absorption.

According to an Example of the present invention, the pharmaceutical composition of the present invention comprises an immediate release phase wherein 80wt% or more Dapoxetine contents thereof are eluted in the eluate within 30 minutes, and a prolonged sustained release phase wherein less than 20wt% Dapoxetine contents thereof are eluted in the eluate within 30 minutes. More preferably, more than 90 wt% of Dapoxetine dissolves in said immediate release phase within 30 minutes, which is because faster exhibition speed of the medicinal effect fulfills patients' satisfaction more due to the relevance of the pharmaceutical composition of the present invention and the improvement of sexual function. Thus, the pharmaceutical compositions of the present invention releases 80-90 wt% or more Dapoxetine content contained in the immediate release phase within 30 minutes from the eluate, thereby to prompt the initial in vivo absorption and exhibit the efficacy of the pharmaceutical compositions of the present invention. In addition, only 10-20wt% release of the Dapoxetine in said prolonged sustained release phase is desirable in the eluate within 30 minutes, and 40-70wt%, most preferably more than 40wt% and less than 50-60wt% release of the Dapoxetine in the total composition including the immediate release phase is desirable. The reason is that the elution under the said range will cause reduced sexual satisfaction of the patients in the early stage of the administration due to the slow rate of exhibition of the medicinal effects; and the too high initial elution will cause increased intended Cmax of Dapoxetine and the resulting side effects due to the over-absorption of Dapoxetine including the Dapoxetine released from the immediate release phase.

In addition, the pharmaceutical composition of the present invention comprises the elution of Dapoxetine over 80wt%, preferably over 90wt% from said prolonged sustained release phase in the eluate during 30 minutes to 10 hours. More preferably, more than 80-90 wt% of Dapoxetine is eluted from said prolonged sustained release phase in the eluate during 1 to 7 hours, or 1 to 3-4 hours. At this time, if the elution of Dapoxetine persists too long, the advantage of the present invention, low potential side effects due to fast elimination rate of blood Dapoxetine, disappears, and the risk of side effects rather increases due to possible drug accumulation in the blood, so too prolonged release time is not desirable.

In the pharmaceutical composition of the present invention, said immediate release phase and prolonged sustained release therein respectively contain 20-80wt% of the entire Dapoxetine contents. Since the effect of Dapoxetine is expressed in proportion to the content size, 30-70wt% of Dapoxetine is preferable in the immediate release phase of the pharmaceutical composition within the range of no side effects, and 40-60wt% is more preferable. In addition, regarding the content of Dapoxetine in the said immediate release phase, 15-100mg is preferable, 20-90mg is more preferable, and 30-60mg is most preferable. It is difficult to obtain the intended medicinal effect with lower Dapoxetine content in the immediate release phase than the aforementioned range, and too much content is not desirable because of high risk of side effects with the SSRI family of drugs, such as vomiting and dizziness.

In addition, the prolonged sustained release phase also includes 20-80wt% of the total Dapoxetine, of which the contents may be adjusted appropriately taking into account of the intended duration to extend the efficacy and the blood peak concentration to cause side effects. At this time, regarding the content of Dapoxetine in the prolonged sustained release phase, 15-100mg is preferable, 20-90mg is more preferable, and 30-60mg is most preferable. In particular, the lower content of Dapoxetine in prolonged sustained release phase is desirable than the absorbed Cmax from the released Dapoxetine of the immediate release phase.

According to a preferred Example of the present invention, the pharmaceutical composition of the present invention can include additional PDE-5 inhibitors in the immediate release phase. As the aforementioned PDE-5 inhibitors, Sildenafil, Tadalafil, Vardenafil, Udenafil, Lodenafil, Mirodenafil, Avanafil, Dasantafil, SLx2101, LAS34179, or mixtures thereof can be used without restriction. The content range of the said PDE-5 inhibitors comprises the normal range currently on the market, including preferable 20-100mg, more preferable 50-100mg of Sildenafil; preferable 5-80mg, more preferable 10-20mg of Tadalafil; 5-40mg of Vardenafil; 50-200mg of Udenafil; 50-200mg of Lodenafil; 20-100mg of Mirodenafil; and 25-300mg of Avanafil. For instance, if Sildenafil or Vardenafil is contained in the immediate release phase as the PDE-5 inhibitor, its contents could be adjusted for the Dapoxetine in the prolonged sustained release phase to dissolve and be absorbed more than 80-90wt% around 3-4 hours. In addition, if Udenafil or Avanafil is contained in the immediate release phase as the PDE-5 inhibitor, its contents could be adjusted for the Dapoxetine in the prolonged sustained release phase to dissolve and be absorbed more than 80-90wt% around 10 hours. Further, if Tadalafil is contained in the immediate release phase as the PDE-5 inhibitor, its content could be adjusted for the Dapoxetine to be eluted for a longer time for the two ingredients to show their medicinal effects to coincide after the pharmaceutical composition of the present invention was taken.

Generally, Dapoxetine has an advantage of less side effect due to the drug accumulation in the body compared to other SSRI family of drugs thanks to its fast rates both in the expression of the effects and elimination from the blood, but also has a downside of too short duration of effect due to the short in vivo primary half life of 1.4 hours. Moreover, if both Dapoxetine and PDE-5 inhibitor are used together there has been inconveniences, due to the difference of the duration of these effects, such as they have to be taken at different times to get the combined effect, or there's a discrepancy of the expression of the effects due to the difference of the duration of the effects when they are taken at the same time. Therefore, the pharmaceutical composition of the present invention is able to match the combination time of Dapoxetine with short duration of effect and PDE-5 inhibitor with a long half-life, by including Dapoxetine both in the immediate release phase and the prolonged sustained release phase.

According to an Example of the present invention, the sustained release phase of the pharmaceutical composition of the present invention may be produced in granules, beads, pellets, dosage form including sustained release coating layer, dosage form containing release retardant, or matrix dosage form; especially the elution time of Dapoxetine can be adjusted under 10-20wt% within the first 30 minutes of elution using delaying method of elution point by adjusting the elution location to intestinal tract with enteric coating or composing inner core with prolonged sustained release phase with core tablets. In addition, the prolonged sustained release phase of the pharmaceutical composition of the present invention can comprise all release dosage form controlled for lower than 10-20wt% of Dapoxetine in the prolonged sustained release phase to be eluted during the first 30 minutes, and more than 80-90wt% of Dapoxetine in the prolonged sustained release phase to be released between 30 minutes and 10 hours. In addition, besides the aforementioned immediate release phase, the pharmaceutical compositions of the present invention can be formulated without restriction in the form of normal tablets, coated tablets, core tablets, multilayer tablets, multi-coated tablets and capsules comprising the prolonged sustained release phase of various forms like granules, beads, pellets, sustained release coating layer, release retardant, or matrix dosage form.

Hereafter, preferable Example examples of dosage forms are explained for manufacturing the pharmaceutical composition of the present invention, but the present invention is not limited thereto.

In the most simple form example for manufacturing a pharmaceutical composition of the present invention, a primary composite was made by mixing Dapoxetine, disintegrating agent, slip modifier and pharmaceutical excipient, and a secondary composite was made by mixing Dapoxetine, hydroxypropyl methylcellulose, ethyl cellulose, polymers like Carbopol, slip modifier and pharmaceutical excipient, followed by direct compression of the composites in a multi-layer tablet press to manufacture multi-layer tablets. On the other hand, the secondary composite is compressed into a core, which is mixed with the primary composite and manufactured to a core tablet in a core tablet press to show dual release. At this time, the core can be coated with sustained release coated layer or enteric coating layer, or release retardant may be included in the core.

In addition, in another example of the pharmaceutical composition of the present invention, immediate release phase and prolonged sustained release phase can be formulated in the form granules to give different release patterns respectively. For example, immediate release granules can be manufactured in wet or dry granulation method using additives such as Dapoxetine, excipients, disintegrating agents or slip modifiers. In addition, granules representing the elution pattern of prolonged sustained release can be prepared either by wet or dry methods after mixing Dapoxetine with polymers, or by additional coating of the formed granules with polymers. In addition, it can be prepared using a fluid bed coater either by spraying polymer binding agents directly into the Dapoxetine, or by spray coating of coating solution containing Dapoxetine onto appropriate particles. Thus prepared prolonged sustained release granules can be additionally mixed and compressed with the immediate release granules or immediate release composites, which were made by simple mixing of Dapoxetine and normal additives, to enable dual release by including the immediate release granules or composites, and prolonged sustained release granules in a tablet or capsule.

On the other hand, the prolonged sustained release granules and immediate release granules or composites can be manufactured in the form of compressed multi-layer tablets split into separate layers. In addition, the dual release can be embodied by first making the core by compression of the prolonged sustained release small granules into tablets, covering the core with the immediate release granules or composites, and compressing them with a core tableting machine.

Coating can be added to the tablets manufactured by the above method. In addition, the prolonged sustained release granules and immediate release granules may be filled in hard capsules to manufacture in the form of capsules.

According to another Example of the present invention, the pharmaceutical composition of the present invention can be manufactured in the form of pellets. For example, the immediate release pellets can be manufactured by first mixing appropriate Dapoxetine-included polymers, such as povidone or hydroxypropyl methylcellulose, with organic solvents, followed by coating them on sugar spheres or starch granules. Additionally, prolonged sustained release pellets can be prepared by coating the said pellets with dissolved mixture of polymers such as ethyl cellulose or Eudragit with appropriate organic solvents. The two types of release can be achieved by filling thus prepared two kinds of pellets in hard capsules. In addition, the two different drug layers can be constructed in a single pellet by first preparing prolonged sustained release pellets in the same manner as above, followed by coating suitable polymer solutions containing a mixture of drugs on the outside of the pellets. Pellets thus prepared can be filled in hard capsules.

According to another Example of the present invention, the present invention can include the matrix form of sustained release phase, which is illustrated in USP5,700,410.

Pharmaceutical composition of the present invention include, without restriction, other types of all dosage forms comprising the two phases, immediate release phase and sustained release phase, in addition to the administrative type as described above; and the release retardants used in each dosage form can comprise, without restriction, all ingredients published in Int'l patent publication No. WO2010/103544 and No.WO2005/094825.

### Advantageous effects

The pharmaceutical composition of the present invention comprises Dapoxetine, which is an agent for treating premature ejaculation, in both the immediate release phase and the prolonged sustained release phase thereof, to thereby immediately exhibit the effectiveness of the pharmaceutical composition of the present invention in order to enable a patient to achieve sexual satisfaction during the early stage of administration, as well as to reduce side effects by means of the time-delayed sustained release of the prolonged sustained release phase during the early stage of administration and enable a continuous in vivo absorption of Dapoxetines, to thereby lengthen the duration of the effectiveness of the pharmaceutical composition of the present invention. Further, agents for treating erectile dysfunction, such as sildenafil, tadalifil or the like can be added to the immediate release phase so as to allow for a coincidence of the durations of the effectiveness of a premature ejaculation treatment agent and erectile dysfunction treatment agents, even though a half-life difference exists between the two types of treatment agents, thus maximizing patient satisfaction.

### Brief description of the drawings

Figure 1 shows the accumulated elution percentage (%) of Dapoxetine, manufactured from Examples and Comparative examples, at each point in time.

Figure 2 shows the amount (mg) of Dapoxetine, manufactured from Examples and Comparative examples, released from each interval.

Figure 3 show the accumulated elution percentage (%) of Dapoxetine in the immediate release phase and sustained release phase.

Figure 4 shows the blood concentration (ng/ml) of Dapoxetine, manufactured from Examples and Comparative examples, at each point in time.

### Best mode for carrying out the invention

The present invention can be detailed by the following examples.

However, the following examples are intended to illustrate the present invention, so the present invention is not limited by the examples below.

Example 1. The manufacture of a pharmaceutical composition (1): dosage form of double-layered tablets

The immediate release composites were prepared by first mixing 33.6g of Dapoxetine HCl, 89.4g of lactose hydrate (Suberb 14SD), 80.0g of microcrystalline cellulose (Avicel pH200) and 12.0g of Crospovidone (Kolidon CL), followed by additional mixing with 1.0g of magnesium stearate, a slip modifier. Apart from this, the prolonged sustained release composites were prepared by first mixing Dapoxetine HCl, 33.6g; Lactose hydrate(Suberb 14SD), 24.4g; hydroxypropyl methylcellulose (Methocel E50), 75.0g; and Kolidon VA64, 35g; followed by additional mixing of Magnesium stearate, 1.0g. The double-layered tablets, comprising a total of 60mg Dapoxetine in each tablet-30mg in each layer-were manufactured by double-layered tablet press compression of 216mg of the immediate release composites and 169mg of the prolonged sustained release composites at each layer respectively in one tablet. Additionally, 15mg of Kollicoat IR White was added per tablet by coating the coating solvent Kollicoat IR White dissolved in purified water.

Example 2. The manufacture of a pharmaceutical composition (1): dosage form of mixture tablets

Mixing was performed by mixing 100.8g of Dapoxetine HCl, 148.2g of Lactose hydrate (Pharmatose 200), 300.0g of microcrystalline cellulose (Avicel pH101) and 36.0g of Croscarmellose sodium (Ac-Di-Sol); followed by binding by the binding agent prepared by dissolving 12.0g of povidone (Kolidon K-30) in purified water; followed by granulation and drying. The immediate release granules were prepared by first spheronization of the above granules in an oscillator and mixing them with 3.0g of magnesium stearate. Apart from this, mixing was performed by mixing 100.8g of Dapoxetine HCl, 316.2g of Lactose hydrate (Pharmatose 200) and 135.0g of hydroxypropyl methylcellulose (Methocel E50); followed by binding by the binding agent prepared by dissolving 15.0g of povidone (Kolidon K-30) in purified water; followed by granulation and drying. The prolonged sustained release granules were prepared after the spheronization of the above granules and mixing them with 3.0g of magnesium stearate. Tablets were prepared that contain 60mg of Dapoxetine per tablet by first mixing the immediate release granules and the prolonged sustained release granules, followed by compressing 390mg of them per tablet using a rotary tablet press. Additionally, 15mg of Kollicoat IR White was added per tablet by coating the coating solvent Kollicoat IR White dissolved in purified water.

Example 3. The manufacture of a pharmaceutical composition (3): dosage form of double-layered tablets

The double-layered tablets, comprising a total of 60mg Dapoxetine in each tablet-30mg in each layer- were manufactured by double-layered tablet press compression of 216mg of the immediate release composites prepared in the above Example 1 and 190mg of the prolonged sustained release granules prepared in the above Example 2 to constitute each layer respectively per tablet. Additionally, 15mg of Kollicoat IR White was added per tablet by coating the coating solvent Kollicoat IR White dissolved in purified water.

Example 4. The manufacture of a pharmaceutical composition (4): dosage form of double-layered tablets

The double-layered tablets, comprising a total of 60mg Dapoxetine in each tablet-30mg in each layer-were manufactured by double-layered tablet press compression of 200mg of the immediate release granules and 190mg of the prolonged sustained release granules prepared in the above Example 2 to constitute each layer respectively per tablet. Additionally, 15mg of Kollicoat IR White was added per tablet by coating the coating solvent Kollicoat IR White dissolved in purified water.

Example 5. The manufacture of a pharmaceutical composition (5): dosage form of hard capsules

200.0g of MicroceLac 100 was fluidized in a fluid bed coater, and was sprayed with the coating solution, which was prepared by dissolving 67.2g of Dapoxetine HCl, 118.0g of hydroxypropyl methylcellulose (Methocel E50) and 16.0g of polyethylene glycol 6000 in methylene chloride-ethanol mixture, to prepare pellets. The prolonged sustained release pellets were prepared by additionally spraying coating solution, which was made by dissolving 40.0g of ethyl cellulose and 10.0g of talc in 75% ethanol solution, to the pellets which were prepared as above. The immediate release layer was prepared by spraying coating solution, which was prepared by dissolving 67.2g of Dapoxetine HCl, 39.8g of hydroxypropyl methylcellulose (Methocel E50) 5.0g of polyethylene glycol 6000 and 4.0g of talc in 75% ethanol, to the pellets prepared as above. The manufactured pellets were filled in hard capsules so as to contain 280mg of pellets (Dapoxetine 60mg) per capsule.

Example 6. The manufacture of a pharmaceutical composition (6): dosage form of hard capsules

200.0g of MicroceLac 100 was fluidized in a fluid bed coater, and was sprayed with the coating solution, which was prepared by dissolving 67.2g of Dapoxetine HCl, 73.8g of hydroxypropyl methylcellulose (Methocel E50), 13.0g of polyethylene glycol 6000 and 6.0g of talc in methylene chloride -ethanol mixture, to prepare immediate release pellets. Apart from this, 200.0g of MicroceLac 100 was fluidized in a fluid bed coater, and was sprayed with the coating solution, which was prepared by dissolving 67.2g of Dapoxetine HCl, 50.8g of hydroxypropyl methylcellulose (Methocel E50) and 12.0g of polyethylene glycol 6000 in methylene chloride - ethanol mixture, to prepare pellets. The prolonged sustained release pellets were prepared by additionally spraying coating solution, which was made by dissolving 60.0g of ethyl cellulose and 10.0g of talc in 75% ethanol solution, to the pellets which were prepared as above. The manufactured immediate release pellets and prolonged sustained release pellets were filled in hard capsules so as to contain 180mg (Dapoxetine 30mg) and 200.0mg (Dapoxetine 30mg) respectively per capsule.

Example 7. The manufacture of a pharmaceutical composition (7): dosage form of mixture tablets

Mixing was performed by mixing 33.6g of Dapoxetine HCl, 49.4g of Lactose hydrate (Pharmatose 200), 100.0g of microcrystalline cellulose (Avicel pH101) and 12.0g of Croscarmellose sodium (Ac-Di-Sol); followed by binding by the binding agent prepared by dissolving 4.0g of povidone (Kolidon K-30) in purified water; followed by granulation and drying. The immediate release granules were prepared by first spheronization of the above granules in an oscillator and mixing them with 1.0g of magnesium stearate. Apart from this, mixing was performed by mixing 67.2g of Dapoxetine HCl, 111.8g of Lactose hydrate (Pharmatose 200) and 45.0g of hydroxypropyl methylcellulose (Methocel E50); followed by binding by the binding agent prepared by dissolving 5.0g of povidone (Kolidon K-30) in purified water; followed by granulation and drying. The prolonged sustained release granules were prepared after the spheronization of the above granules and mixing them with 1.0g of magnesium stearate. Tablets were prepared that contain 200mg of immediate release granules(30mg of Dapoxetine) and 230mg of prolonged sustained release granules(60mg of Dapoxetine) in separate layers per tablet by compression using a double-layer tablet press. Additionally, 15mg of Kollicoat IR White was added per tablet by coating the coating solvent Kollicoat IR White dissolved in purified water.

Example 8. The manufacture of a pharmaceutical composition (8): dosage form of mixture tablets

Mixing was performed by mixing 33.6g of Dapoxetine HCl, 10.0g of Tadalafil, 49.4g of Lactose hydrate (Pharmatose 200), 90.0g of microcrystalline cellulose (Avicel pH101) and 12.0g of Croscarmellose sodium (Ac-Di-Sol); followed by binding by the binding agent prepared by dissolving 4.0g of povidone (Kolidon K-30) in purified water; followed by granulation and drying. The immediate release granules were prepared by first spheronization of the above granules in an oscillator and mixing them with 1.0g of magnesium stearate. Apart from this, mixing was performed by mixing 33.6g of Dapoxetine HCl, 105.4g of Lactose hydrate (Pharmatose 200) and 55.0g of hydroxypropyl methylcellulose (Methocel E50); followed by binding by the binding agent prepared by dissolving 5.0g of povidone (Kolidon K-30) in purified water; followed by granulation and drying. The prolonged sustained release granules were prepared after the spheronization of the above granules and mixing them with 1.0g of magnesium stearate. The double-layered tablets, comprising a total of 60mg Dapoxetine in each tablet-30mg in each layer-were manufactured by double-layered tablet press compression of 200mg of the immediate release granules and 200mg of the prolonged sustained release granules at each layer respectively in one tablet. Additionally, 15mg of Kollicoat IR White was added per tablet by coating the coating solvent Kollicoat IR White dissolved in purified water.

Example 9. The manufacture of a pharmaceutical composition (9): dosage form of mixture tablets

16.8g of Dapoxetine HCl was mixed with 35.12g of Sildenafil citrate, 41.99g of Lactose hydrate (Supertab 14SD), 25.0g of microcrystalline cellulose (Vivapur 12), and 4.35g of Croscarmellose sodium (Ac-Di-Sol). After that, the immediate release composites were prepared by screening (through 40mesh), adding and mixing 0.5g of colloidal silicon dioxide (Aerosil 200) and 1.25g of magnesium stearate. Apart from this 16.8g of Dapoxetine HCl was mixed with 14.3g of Lactose hydrate (Supertab 14SD), 7.5g of microcrystalline cellulose (Vivapur 12) and 60.0g of hydroxypropyl methylcellulose (Pharmacoat 606). After that, the prolonged sustained release composites were prepared by screening (through 40mesh), adding and mixing 0.4g of colloidal silicon dioxide (Aerosil 200) and 1.0g of magnesium stearate. The double-layered tablets, comprising 30mg Dapoxetine and 50mg of Sildenafil in the immediate release layer and 30mg Dapoxetine in the prolonged sustained release layer in each tablet, were manufactured by double-layered tablet press compression of 250mg of the immediate release granules and 200mg of the prolonged sustained release granules at each layer respectively in one tablet. Additionally, 15mg of Kollicoat IR White was added per tablet by coating the coating solvent Kollicoat IR White dissolved in purified water.

Example 10. The manufacture of a pharmaceutical composition (10): dosage form of mixture tablets

16.8g of Dapoxetine HCl was mixed with 35.12g of Sildenafil citrate, 41.99g of Lactose hydrate (Supertab 14SD), 25.0g of microcrystalline cellulose (Vivapur 12), and 4.35g of Croscarmellose sodium (Ac-Di-Sol). After that, the immediate release composites were prepared by screening (through 40mesh), adding and mixing 0.5g of colloidal silicon dioxide (Aerosil 200) and 1.25g of magnesium stearate. Apart from this 33.6g of Dapoxetine HCl was mixed with 16.8g of Lactose hydrate (Supertab 14SD), 7.5g of microcrystalline cellulose (Vivapur 12) and 90.0g of hydroxypropyl methylcellulose (Pharmacoat 606). After that, the prolonged sustained release composites were prepared by screening (through 40mesh), adding and mixing 0.6g of colloidal silicon dioxide (Aerosil 200) and 1.5g of magnesium stearate. The double-layered tablets, comprising 30mg Dapoxetine and 50mg of Sildenafil in the immediate release layer and 60mg Dapoxetine in the prolonged sustained release layer in each tablet, were manufactured by double-layered tablet press compression of 250mg of the immediate release granules and 300mg of the prolonged sustained release granules at each layer respectively in one tablet. Additionally, 15mg of Kollicoat IR White was added per tablet by coating the coating solvent Kollicoat IR White dissolved in purified water.

Comparative Example 1. The manufacture of a pharmaceutical composition (11): single tablets

Mixing was performed by mixing 33.6g of Dapoxetine HCl, 140.4g of Lactose hydrate (Pharmatose 200), 100.0g of microcrystalline cellulose (Avicel pH101) and 16.0g of Croscarmellose sodium (Ac-Di-Sol); followed by binding by the binding agent prepared by dissolving 7.0g of povidone (Kolidon K-30) in purified water; followed by granulation and drying. Tablets, containing 30mg of Dapoxetine, were prepared by first spheronization of the above granules in an oscillator, mixing them with 3.0g of magnesium stearate, and compression of 300mg per tablet in a rotary tablet press. Additionally, 15mg of Kollicoat IR White was added per tablet by coating the coating solvent Kollicoat IR White dissolved in purified water.

Comparative Example 2. The manufacture of a pharmaceutical composition (12): single tablets

Mixing was performed by mixing 67.2g of Dapoxetine HCl, 136.8g of Lactose hydrate (Pharmatose 200), 100.0g of microcrystalline cellulose (Avicel pH101) and 16.0g of Croscarmellose sodium (Ac-Di-Sol); followed by binding by the binding agent prepared by dissolving 7.0g of povidone (Kolidon K-30) in purified water; followed by granulation and drying. Tablets, containing 60mg of Dapoxetine, were prepared by first spheronization of the above granules in an oscillator, mixing them with 3.0g of magnesium stearate, and compression of 330mg per tablet in a rotary tablet press. Additionally, 15mg of Kollicoat IR White was added per tablet by coating the coating solvent Kollicoat IR White dissolved in purified water.

Comparative Example 3. The manufacture of a pharmaceutical composition (13): single tablets

33.6g of Dapoxetine HCl was mixed with 35.12g of Sildenafil citrate, 100.19g of Lactose hydrate (Supertab 14SD), 50.0g of microcrystalline cellulose (Vivapur 12), and 4.35g of Croscarmellose sodium (Ac-Di-Sol). After that, 0.5g of colloidal silicon dioxide (Aerosil 200) and 1.25g of magnesium stearate were screened (through 40mesh), added and mixed. Tablets, comprising 60mg Dapoxetine and 50mg of Sildenafil, were manufactured by rotary tablet press compression of 450mg of the above mixture. Additionally, 15mg of Kollicoat IR White was added per tablet by coating the coating solvent Kollicoat IR White dissolved in purified water.

Comparative Example 4. The manufacture of a pharmaceutical composition (14): single tablets

Tablets, comprising 45mg (75%) from the immediate release granules and 15mg (25%) from the prolonged sustained release granules per tablet, were manufactured by mixing and rotary tablet press compression of 300g of the immediate release granules and 95g of the prolonged sustained release granules prepared in the above Example 2 to constitute 395mg of weight per tablet. Additionally, 15mg of Kollicoat IR White was added per tablet by coating the coating solvent Kollicoat IR White dissolved in purified water.

Comparative Example 5. The manufacture of a pharmaceutical composition (15): separate tablets

Dapoxetine immediate release tablets (30 mg of Dapoxetine contained per tablet) and Dapoxetine prolonged sustained release tablets (30 mg of Dapoxetine contained per tablet) were manufactured with immediate release granules and prolonged sustained release granules which were prepared for the above Example 4. Additionally, 7mg of Kollicoat IR White was added per tablet by coating the coating solvent Kollicoat IR White dissolved in purified water.

Experimental Example 1. Dissolution test of the active ingredients

The elution was performed, for one tablet each that was respectively prepared in each Example and Comparative Example, in accordance with USP Dissolution Apparatus 2 - Paddle, the 2nd dissolution test methodology, using in 900ml of 0.1 M HCl and at 50rpm rotation. The fluid collected at each time point was filtrated by 0.45µm membrane filter, and tested per liquid chromatography to determine the concentration of Dapoxetine at each point of time; the cumulative dissolution rate (%) at each point of time and the amount (mg) Dapoxetine released in each segment were shown in a graph.

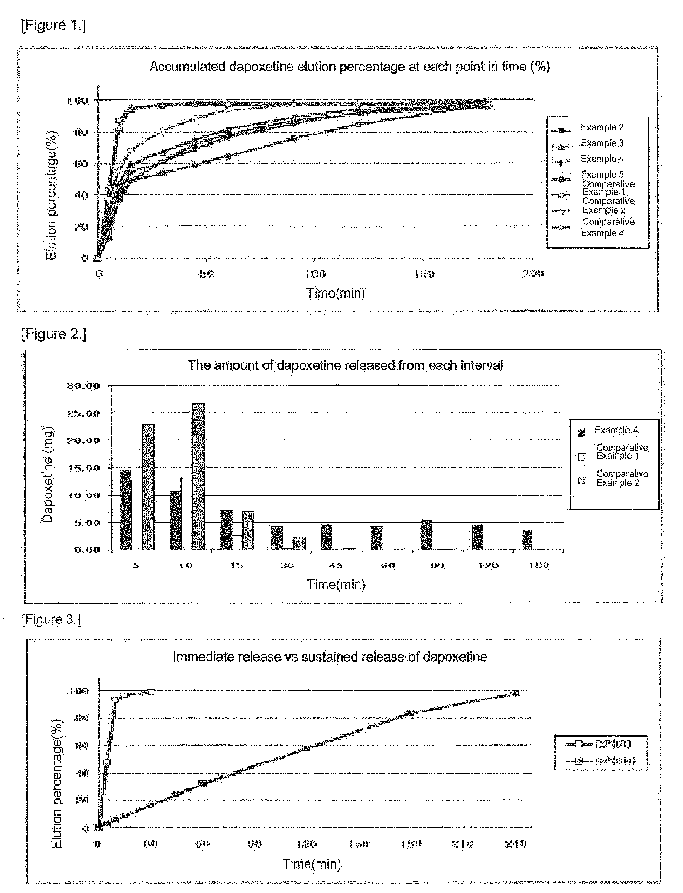

As a result, in the case of the pharmaceutical composition, which was made with Dapoxetine HCl as the general immediate release formulation, in Comparative Example 1 and Comparative Example 2, more than 90% of the administered drug was released within the initial 15 minutes, and the subsequent release of the drug was negligible; however, approximately 80% of elution was occurred in the initial 30 minutes for the pharmaceutical compositions of Comparative Example 4. By contrast, for the pharmaceutical composition of Examples 1 and 5, it was confirmed that 50-70% of the entire drug (i.e., corresponds to 30mg of Dapoxetine) was released fast, and the remaining 30-50% of the drug was then released slowly over the 120-180 minutes (Fig. 1 and 2).

Experimental Example 2. Dissolution test of the active ingredients

A dissolution test was performed in the same method as in the above Experimental Example 1, using the immediate release tablets and the prolonged sustained release tablets prepared in the above Comparative Example 5.

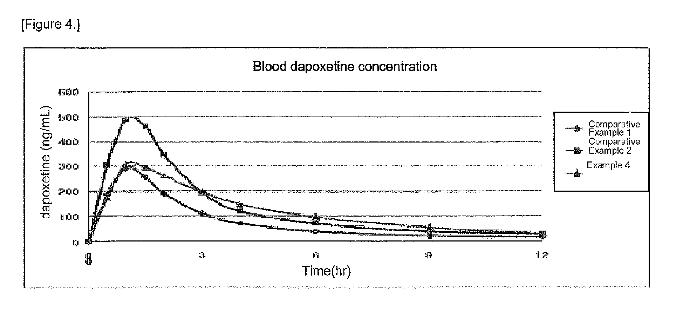

As a result, it was confirmed that 85% or more Dapoxetine of the immediate release tablet was eluted in the first 30 minutes; and less than 20% of Dapoxetine of the prolonged sustained release tablet was eluted in the first 30 minutes, and 90% or more in 4 hours (Fig.4).

Experimental Example 3. Blood concentration measurement of the active ingredients

Plasma concentrations of 10 volunteers were measured after a single oral dose of each table prepared as in the above Example 4, Comparative Examples 1 and 2.

As a result, in the case of tablets prepared in Example 4, it was found that the effect expression occurred quickly due to the fast elution of the immediate release phase as in the case of Comparative Examples 1 and 2. In addition, it was confirmed that the efficacy lasting can be extended as well as the side effects can be significantly reduced, compared to Comparative Example 2 of the same content, by controlling the rate of release and the content of the prolonged sustained release phase (Fig. 4).

Experimental Example 4. Sensory evaluation of the pharmaceutical composition

The efficacy of the pharmaceutical composition of the present invention was assessed, for 20 patients over the age of 20 with premature ejaculation and erectile dysfunction, using the tablets manufactured in the above Examples 4, 7, 9 and 10, and Comparative Examples 2 and 3. For this purpose, the subjects were administered the pharmaceutical composition of the present invention 2,4,6 or 8 hours prior to anticipated sexual activity. The validity of the pharmaceutical composition of the present invention was determined by the percentage of items after evaluation of overall satisfaction questions (refer to Korean Patent Registration No. 719977 or WO2001/1751); the results are shown in Table 1 below...

- Much better

- Better

- A little better

- Same

- A little worse

- Worse

- Much worse

Table 1

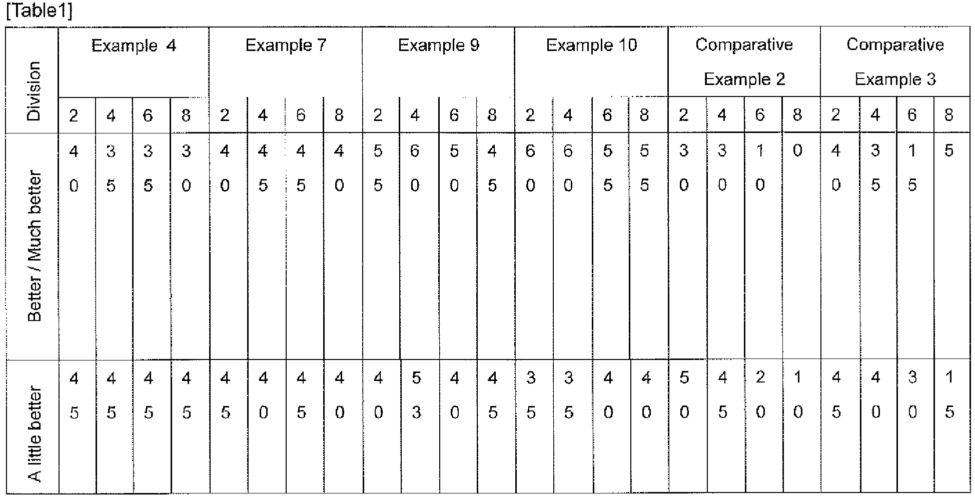

As a result, the pharmaceutical compositions of the present invention showed high ratio of at least 80% of 'a little better', 'better' or 'much better' when administered 2-8 hours before sexual activity. In particular, in the case of the pharmaceutical composition, in Examples 9 and 10, which contain sildenafil citrate in the immediate release layer, showed the ratio of at least 90% or more, in most cases 95%, meaning significantly higher patient satisfaction. In contrast, in the case of the pharmaceutical composition, of Comparative Examples 2 and 3, containing only Dapoxetine, or in combination with sildenafil citrate in a single tablet, it was confirmed that a relatively high level of satisfaction shown when administered 2 hours before sexual activity, but less than 50% of satisfaction is shown when administered 6-8 hours before, so the medicinal efficacy lasts very short of the pharmaceutical composition of the present invention.

Experimental Example 5. Side effects of the pharmaceutical composition

The volunteers, who were administered the pharmaceutical composition of the present invention of the above Experimental Example 4, were observed for any medical side effects.

As a result, no side effects occurred in most of the volunteers, or if any, very mild side effects such as diarrhea, dizziness, vomiting, and headache occurred. In addition, with increasing content of the active ingredient of a pharmaceutical composition contained within, and when the initial dissolution content exceeds 70 wt% as in the case of Comparative Example 4, it showed a tendency of a slight increase in side effects symptoms (Table 2).

Table 2

**[Table 2]**

| Side effects [n (%)] | Example 4 | Example 7 | Example 9 | Comparative Example 1 | Comparative Example 2 | Comparative Example 3 | Comparative Example 4 |
|---|---|---|---|---|---|---|---|
| Diarrhea | 2(10) | 2(10) | 2(10) | 2(10) | 4(20) | 5(25) | 3(15) |
| Dizziness | 1(5) | 2(10) | 1(5) | 2(10) | 3(15) | 4(20) | 3(15) |
| Vomiting | 1(5) | 2(10) | 1(5) | 1(5) | 2(10) | 2(10) | 2(10) |
| Headache | 0(0) | 1(5) | 0(0) | 1(5) | 1(5) | 1(5) | 1(5) |

## Claims

1. A time-delayed sustained release pharmaceutical composition for oral administration, which is comprised of an immediate release phase and a prolonged sustained release phase,
wherein said immediate release phase and prolonged sustained release phase respectively contain Dapoxetine therein as an active ingredient;
**characterized in that** Dapoxetine contained in said immediate release phase is eluted 80wt% or more from an eluate in 30 minutes, and Dapoxetine contained in said prolonged sustained release phase is eluted less than 20wt% from the eluate during the first 30 minutes.

2. A pharmaceutical composition according to Claim 1,
**characterized in that** between 40wt% and 70 wt% of the total contents of Dapoxetine thereof is eluted during the first 30 minutes of elution.

3. A pharmaceutical composition according to Claim 1,
**characterized in that** the respective contents of Dapoxetine in said immediate release phase and prolonged sustained release phase are 20-80wt% of the total contents.

4. A pharmaceutical composition according to Claim 3,
**characterized in that** the each contents of Dapoxetine in said immediate release phase and prolonged sustained release phase are 40-60wt% of the total contents respectively.

5. A pharmaceutical composition according to Claim 1,
**characterized in that** the each contents of Dapoxetine in said immediate release phase and prolonged sustained release phase are 15-100mg respectively.

6. A pharmaceutical composition according to Claim 5,
**characterized in that** the each contents of Dapoxetine in said immediate release phase and prolonged sustained release phase are 30-60mg respectively.

7. A pharmaceutical composition according to Claim 1,
**characterized in that** said sustained release phase may be produced in a dosage form selected from the group comprising granules, beads, pellets, dosage form including sustained release coating layer, or matrix dosage form.

8. A pharmaceutical composition according to Claim 1,
**characterized in that** more than 80 wt% of Dapoxetine in the said prolonged sustained release phase is eluted during 30 minutes to 10 hours of the said prolonged sustained release phase.

9. A pharmaceutical composition according to Claim 1,
**characterized in that** the said prolonged sustained release phase is embodied by enteric coating or the core of the core tablets.

10. A pharmaceutical composition according to Claim 1,
**characterized in that** the pharmaceutical composition has a dosage form selected from the group comprising normal tablets, coated tablets, core tablets, multilayer tablets, multi-coated tablets and capsules.

11. A pharmaceutical composition according to one of Claim 1-10,
**characterized in that** the said immediate release phase of the said pharmaceutical composition additionally comprises Phosphodiesterase-5 (PDE-5) inhibitors.

12. A pharmaceutical composition according to Claim 11,
**characterized in that** the said Phosphodiesterase-5 (PDE-5) can be selected from the group comprising Sildenafil, Tadalafil, Vardenafil, Udenafil, Lodenafil, Mirodenafil, Avanafil, Dasantafil, SLx2101, LAS34179, or mixtures thereof.

13. A pharmaceutical composition according to Claim 12,
**characterized in that** the contents of the said Sildenafil is in the range of 20-100mg.

14. A pharmaceutical composition according to Claim 13,
**characterized in that** the said immediate release phase comprises 30mg of Dapoxetine and 50mg of Sildenafil, and the said prolonged sustained release phase comprises 30-60mg of Dapoxetine.

15. A pharmaceutical composition according to Claim 12,
**characterized in that** the contents of the said Tadalafil is in the range of 5-80mg.

16. A pharmaceutical composition according to Claim 15,
**characterized in that** the said immediate release phase comprises 30mg of Dapoxetine and 10-20mg of Tadalafil, and the said prolonged sustained release phase comprises 30-60mg of Dapoxetine.
